# EUROPEAN PATENT APPLICATION

(11) **EP 4 785 870 A1**
(43) Date of publication of application: **05.08.2026**
(21) Application number: 24872391.8
(22) Date of filing: 26.09.2024
(51) Int. Cl.: A61B 6/51, A61B 6/02, A61C 19/04, A61C 19/05

(54) **DATA GENERATION PROGRAM, DATA GENERATION METHOD, DATA GENERATION DEVICE, AND X-RAY IMAGING DEVICE**

(30) Priority: 28.09.2023 JP 2023167779
(71) Applicant: J. MORITA MANUFACTURING CORPORATION, Fushimi-ku, Kyoto-shi, Kyoto 6128533 (JP)
(72) Inventor: YOSHIKAWA, Hideki, Kyoto-shi, Kyoto 612-8533 (JP)
(74) Representative: Müller Hoffmann & Partner
(86) International application number: PCT/JP2024/034417
(87) International publication number: WO 2025/070606

(57) **Abstract**

A data generation program is configured to cause a computing device to perform a display step of showing on a display, an oral cavity image three-dimensionally showing an oral cavity including a tooth, an image superimposition step of superimposing a virtual surface on the oral cavity image based on a user input or machine learning, and an image change step of changing the oral cavity image in accordance with a distance between the virtual surface and an occlusal surface of the tooth shown in the oral cavity image.

## Description

### TECHNICAL FIELD

The present disclosure relates to a data generation program, a data generation method, a data generation apparatus, and X-ray equipment configured to generate image data for checking a state of a tooth.

### BACKGROUND ART

It is important for a patient to form and keep an ideal occlusal condition of a tooth. The occlusal condition is generally checked with the use of articulating paper or a dental model or with digital data obtained by an intraoral scanner (IOS). PTL 1 discloses a dental occlusal stress measurement apparatus including a sensor sheet to which an imprint material is applied, the sensor sheet being to be bitten by a subject, and a computer configured to process data indicating a stress detection point in the sensor sheet at the time of occlusion. The dental occlusal stress measurement apparatus can detect a premature contact position by detection of a position of contact of a tooth and occlusal stress with the sensor sheet.

### CITATION LIST

### PATENT LITERATURE

PTL 1: Japanese Patent Laying-Open No. 2005-279094

### SUMMARY OF INVENTION

### TECHNICAL PROBLEM

According to the dental occlusal stress measurement apparatus disclosed in PTL 1, the premature contact position can be detected with the use of the sensor sheet. Depending on a degree of bite of the sensor sheet by the subject, however, the position of contact of the tooth and the occlusal stress may vary and the occlusal condition may not highly accurately be checked. In addition, the conventional checking method described above is limited to presentation of the current occlusal condition of a patient, and an operator has been unable to highly accurately check the premature contact position of each tooth.

The present disclosure was made to solve such a problem, and an object thereof is to provide a technique that allows highly accurate checking of an occlusal condition of a tooth.

### SOLUTION TO PROBLEM

According to one example in the present disclosure, a data generation program configured to generate image data for checking a state of a tooth is provided. The data generation program is configured to cause a computer to perform a display step of showing on a display, an oral cavity image three-dimensionally showing an oral cavity including the tooth, an image superimposition step of superimposing a virtual surface on the oral cavity image based on a user input or machine learning, and an image change step of changing the oral cavity image in accordance with a distance between the virtual surface and an occlusal surface of the tooth shown in the oral cavity image.

According to one example in the present disclosure, a data generation method of generating image data for checking a state of a tooth is provided. The data generation method includes, as processing to be performed by a computer, a display step of showing on a display, an oral cavity image three-dimensionally showing an oral cavity including the tooth, an image superimposition step of superimposing a virtual surface on the oral cavity image based on a user input or machine learning, and an image change step of changing the oral cavity image in accordance with a distance between the virtual surface and an occlusal surface of the tooth shown in the oral cavity image.

According to one example in the present disclosure, a data generation apparatus that generates image data for checking a state of a tooth is provided. The data generation apparatus includes a display that shows an image, an input device that receives a user input, and a control device that controls the display based on the user input. The control device is configured to cause the display to show an oral cavity image three-dimensionally showing an oral cavity including the tooth, to superimpose a virtual surface on the oral cavity image based on the user input or machine learning, and to change the oral cavity image in accordance with a distance between the virtual surface and an occlusal surface of the tooth shown in the oral cavity image.

According to one example in the present disclosure, X-ray equipment that generates image data for checking a state of a tooth is provided. The X-ray equipment includes an imaging unit configured to image an oral cavity including the tooth, a display configured to show an image, an input device configured to receive a user input, and a control device configured to control the display based on the user input. The control device is configured to cause the display to show an oral cavity image three-dimensionally showing the oral cavity imaged by the imaging unit, to superimpose a virtual surface on the oral cavity image based on the user input or machine learning, and to change the oral cavity image in accordance with a distance between the virtual surface and an occlusal surface of the tooth shown in the oral cavity image.

### ADVANTAGEOUS EFFECTS OF INVENTION

According to the present disclosure, since the virtual surface can be superimposed on the oral cavity image shown on the display and the oral cavity image can be changed in accordance with the distance between the virtual surface and the occlusal surface of the tooth shown in the oral cavity image, the user can highly accurately check the occlusal condition of the tooth.

### BRIEF DESCRIPTION OF DRAWINGS

Fig. 1 is a diagram showing an application of a data generation apparatus according to a first embodiment.
Fig. 2 is a block diagram showing a configuration of the data generation apparatus according to the first embodiment.
Fig. 3 is a diagram for illustrating an exemplary virtual plane set in an oral cavity image.
Fig. 4 is a diagram for illustrating an exemplary virtual plane set in an oral cavity image.
Fig. 5 is a diagram for illustrating exemplary calculation of a Balkwill angle.
Fig. 6 is a diagram for illustrating exemplary change of a heat map in the oral cavity image with movement of the virtual plane.
Fig. 7 is a diagram for illustrating exemplary change of a heat map in the oral cavity image with movement of the virtual plane.
Fig. 8 is a diagram for illustrating an example of a dentition in a maxilla and a dentition in a mandible to which a heat map is annexed.
Fig. 9 is a flowchart for illustrating exemplary data generation processing performed by the data generation apparatus according to the first embodiment.
Fig. 10 is a flowchart for illustrating exemplary Balkwill angle determination processing performed by the data generation apparatus according to the first embodiment.
Fig. 11 is a diagram for illustrating an exemplary sphere of Monson set in the oral cavity image.
Fig. 12 is a diagram for illustrating exemplary change of the heat map in the oral cavity image with movement of a virtual curved surface.
Fig. 13 is a diagram for illustrating exemplary change of the heat map in the oral cavity image with movement of a virtual curved surface.

### DESCRIPTION OF EMBODIMENTS

### <First Embodiment>

A first embodiment of the present disclosure will be described in detail with reference to the drawings. The same or corresponding elements in the drawings have the same reference characters allotted and description thereof will not be repeated.

### [Application]

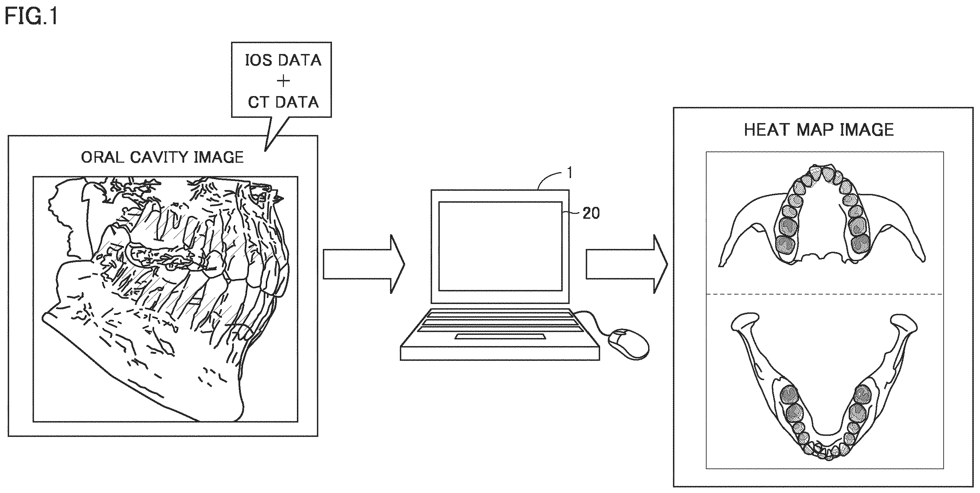

An application of a data generation apparatus 1 according to the first embodiment will be described with reference to Fig. 1. Fig. 1 is a diagram showing an application of data generation apparatus 1 according to the first embodiment. In a preparation stage, a user of data generation apparatus 1 obtains three-dimensional data (optical scanner data) including position information of each point in a point group (a plurality of points) indicating a surface of a biological tissue including a tooth and a gingiva in an oral cavity by scanning the inside of the oral cavity of a subject with the use of a not-shown three-dimensional scanner (optical scanner). The three-dimensional data includes as position information, a coordinate (X, Y, Z) of each point indicating the surface of the biological tissue in an X-axis direction, a Y-axis direction, and a Z-axis direction that are predetermined. Directions in the position information described above may be, for example, such that the X-axis direction is a lateral direction (for example, a left-right direction of the head), the Y-axis direction is a longitudinal direction (for example, a front-rear direction of the head), and the Z-axis direction is a height direction (for example, a direction of extension of a body axis of the head of a human body in an upright position). For example, with the center of the head being defined as a reference point (for example, where a numerical value is zero) of a coordinate, a right side of the reference point may be set as a +X direction (positive direction) of an X axis, a left side of the reference point may be defined as a -X direction (negative direction) of the X axis, a front side of the reference point may be set as a +Y direction (positive direction) of a Y axis, a rear side of the reference point may be defined as a -Y direction (negative direction) of the Y axis, an upper side of the reference point may be set as a +Z direction (positive direction) of a Z axis, and a lower side of the reference point may be defined as a -Z direction (negative direction) of the Z axis. Along each of the X axis, the Y axis, and the Z axis, a direction from a side smaller in numerical value toward a side larger in numerical value is a + direction and a direction from the side larger in numerical value toward the side smaller in numerical value is a -direction. For example, with reference to the X axis, a direction from a -X side toward a +X side is the +X direction and a direction from the +X side toward the -X side is the -X direction. Furthermore, the three-dimensional data may include color information indicating an actual color of a portion (a surface portion of the biological tissue) corresponding to each point in the point group (the plurality of points) indicating the surface of the biological tissue including the tooth and the gingiva in the oral cavity. Regarding a "tooth axis" which will be described later, for a tooth in a mandible, a tooth in a maxilla is considered as a "tooth on an opposite side," and for a tooth in the maxilla, a tooth in the mandible is considered as a "tooth on the opposite side." A side of the "tooth on the opposite side" along the tooth axis of a certain tooth is also referred to as "upward along the tooth axis" and a side distant from the "tooth on the opposite side" along the tooth axis of the certain tooth is also referred to as "downward along the tooth axis."

The "user" includes an operator (a doctor or the like) or an assistant (a dental nurse, a dental technician, a nurse, or the like) in various fields such as dentistry, dental surgery, orthopedics, plastic surgery, and cosmetic surgery. The "subject" includes a patient of dentistry, dental surgery, orthopedics, plastic surgery, and cosmetic surgery. An expression "a user does something" below encompasses an example in which a user gives an execution command to have an apparatus or a program perform processing or operate.

The three-dimensional scanner is what is called an intraoral scanner capable of optically picking up an image of the inside of the oral cavity of a subject by a confocal method or triangulation, and it is capable of obtaining position information of each point in the point group that defines the surface of the biological tissue (for example, the tooth and the gingiva in the oral cavity) set in a certain coordinate space that is to be scanned. Optical imaging and scanning of the inside of the oral cavity is also referred to as intraoral scan (IOS). The user can generate a rendered image (an appearance image) showing a three-dimensional geometry of the biological tissue based on three-dimensional data obtained by the three-dimensional scanner. The "rendered image" is an image generated by processing or edition of certain data. For example, the user can generate a rendered image showing a biological tissue (a part of the biological tissue that can be shown based on IOS data) viewed from a prescribed point of view by processing or edition of three-dimensional data of the biological tissue obtained by the three-dimensional scanner. Furthermore, the user can generate, by varying the prescribed point of view in multiple directions, a plurality of rendered images showing the biological tissue viewed in multiple directions. The rendered image may be a two-dimensional image. The two-dimensional image may be an image obtained by projection of image data on a projection plane for coordinate operation that intersects in a direction of line of sight. For example, the rendered image may be generated as a three-dimensional image that can stereoscopically be observed with a viewer such as general-purpose VR goggles.

The user can also obtain three-dimensional volume (voxel) data of a hard tissue portion (a bone, a tooth, or the like) around a maxilla and a mandible of a subject by imaging the maxilla and the mandible of the subject with a not-shown computed tomography (CT) imager. The CT imager is "X-ray equipment" that takes a CT of the maxilla and the mandible of the subject by rotation around the face of the subject, of a transmitter and a receiver of X rays which are a kind of radioactive rays. The user can generate the rendered image (a CT image or an appearance image) showing the three-dimensional geometry of the biological tissue based on volume data obtained by the CT imager, of the biological tissue which is an imaging target. For example, the user can generate a rendered image showing the biological tissue (a part of the biological tissue that can be shown based on the CT data) viewed from a prescribed point of view by processing or edition of volume data of the biological tissue obtained by the CT imager. Furthermore, the user can generate, by varying the prescribed point of view in multiple directions, a plurality of rendered images showing the biological tissue viewed from multiple directions. Similarly to the case of the IOS data, the rendered image may be a two-dimensional image or a three-dimensional image.

The three-dimensional data including position information of each point in a point group indicating the surface of the biological tissue, the position information being obtained by the three-dimensional scanner, is also referred to as "IOS data," and the rendered image generated based on the IOS data is also referred to as an "IOS image" below. The three-dimensional volume data obtained by the CT imager is also referred to as "CT data" and the rendered image generated based on the CT data is also referred to as a "CT image." The IOS image can show in a very detailed manner, a surface geometry of the biological tissue to be scanned, whereas it cannot show an internal construction (an alveolar bone, a root apex portion, or the like) that does not appear at the surface of the biological tissue. The CT image can show the hard tissue portion (the bone, the tooth, or the like) of an imaging target relatively in detail, whereas it cannot show a soft tissue portion (the skin, the gingiva, or the like) in a manner as detailed as the hard tissue portion.

The user can generate oral cavity image data by combining the IOS data and the CT data obtained for the same subject. The IOS data and the CT data are different from each other in data format. Therefore, the user generates the oral cavity image data which is combination of the IOS data and the CT data, for example, by converting the data format of the IOS data into the data format of the CT data and subjecting the three-dimensional geometry of the surface of the biological tissue to pattern matching with the use of converted data of both of them. Alternatively, the user may generate the oral cavity image data by converting the data format of the CT data into the data format of the IOS data and subjecting the three-dimensional geometry of the surface of the biological tissue to pattern matching with the use of converted data of both of them. Alternatively, the user may generate the oral cavity image data by converting the data formats of the CT data and the IOS data into a common data format and subjecting the three-dimensional geometry of the surface of the biological tissue to pattern matching with the use of converted data of both of them. How to process a data format may be determined on a program side.

The user can generate a rendered image (for example, an oral cavity image shown in Fig. 1) showing a two-dimensional biological tissue (a part of the biological tissue that can be shown based on both of the IOS data and the CT data) viewed from a prescribed point of view by processing or edition of the oral cavity image data. As shown in Fig. 1, the oral cavity image can three-dimensionally show the surface geometry of the biological tissue based on the IOS data and a tomographic structure or an appearance in the hard tissue portion (the bone, the tooth, or the like) based on the CT data. In generation of the oral cavity image, the user may adjust a luminance, a contrast, a transmittance, and the like as necessary in each of the IOS data and the CT data.

Data generation apparatus 1 may obtain the IOS data from the three-dimensional scanner, obtain the CT data from the CT imager, and generate the oral cavity image data based on the obtained IOS data and CT data in accordance with a user input. Alternatively, data generation apparatus 1 may obtain from another apparatus, the oral cavity image data generated by the user with another apparatus, without obtaining the IOS data and the CT data.

The oral cavity image shows based on the CT data, the three-dimensional geometry of the hard tissue portion such as the alveolar bone and the root apex portion and shows based on the IOS data, the three-dimensional geometry of the soft tissue portion such as the gingiva that cannot be shown based on the CT data. The oral cavity image can thus show in detail also the soft tissue portion such as the gingiva that cannot be shown only based on the CT data, owing to supplement by the IOS data, together with the hard tissue portion such as the alveolar bone and the root apex portion.

As shown in Fig. 1, data generation apparatus 1 shows on a display 20, an oral cavity image showing the oral cavity including teeth. Data generation apparatus 1 can show on display 20, the oral cavity image showing the oral cavity viewed from various points of view by performing processing on the oral cavity image shown on display 20, based on a user input. Furthermore, data generation apparatus 1 can add a heat map showing a premature contact position or the like to at least one occlusal surface in a dentition in the maxilla and a dentition in the mandible in the oral cavity image shown on display 20. The user can thus check the occlusal condition of each tooth by checking the occlusal surface in heat map representation. The "heat map" refers to an image which shows a difference between a value at a certain portion and a value at another portion with a degree of change in hue. For example, for the heat map, processing for changing stepwise, an image from a warm color to a cold color in the descending order of values in accordance with a distance between the virtual plane and the occlusal surface may be adopted. For the heat map, processing for changing stepwise, an image from a cold color to a warm color in the descending order of values in accordance with the distance between the virtual plane and the occlusal surface may be adopted.

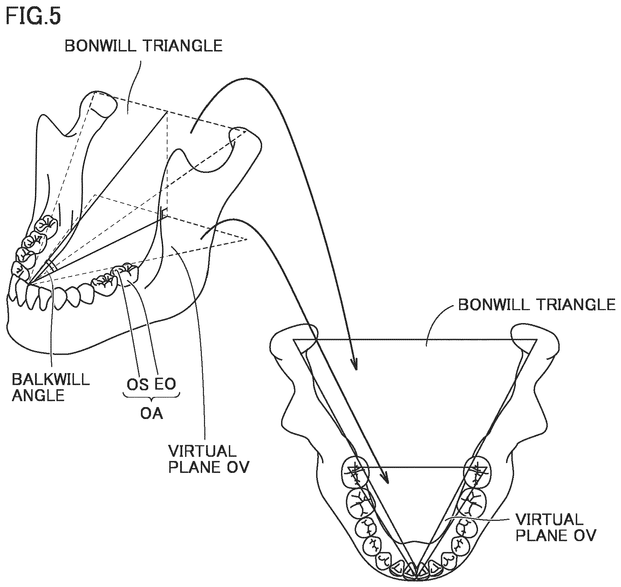

In a narrow sense, the "occlusal surface" refers to a surface where, when a tooth in the maxilla and a tooth in the mandible meet each other, paired teeth come in contact with each other, and it refers to a surface where food is crushed and ground when one eats food. In the embodiment, the occlusal surface in such a sense is expressed as an "occlusal surface OS." Though an area to which a heat map is to be added may be occlusal surface OS, the area may include not only occlusal surface OS but also an area EO contiguous to occlusal surface OS. Such an area in a tooth surface where the heat map is to be added is also referred to as an "occlusal surface defining area OA" (see Fig. 5 which will be described later). Naturally, since an area of a tooth which is not a molar tooth, such as an area of a foretooth, includes an occlusal portion, such an area may be encompassed in an area covered by occlusal surface defining area OA. In an example where the "occlusal surface" in the embodiment refers to the area to which the heat map is to be added, the "occlusal surface" may mean occlusal surface defining area OA. The "occlusal surface" which means occlusal surface defining area OA is also referred to as an "occlusal surface OA" below.

Data generation apparatus 1 is not limited to the data generation apparatus configured to generate the oral cavity image based on combined data of the IOS data and the CT data. For example, data generation apparatus 1 may be configured to generate the oral cavity image only based on the IOS data. The oral cavity image generated only based on the IOS data can at least show a surface geometry of the tooth and the gingiva in the oral cavity. Alternatively, data generation apparatus 1 may be configured to generate the oral cavity image only based on the CT data. The oral cavity image generated only based on the CT data can show at least a geometry of the hard tissue portion such as the tooth and the bone. Since an image of the surface of the tooth can be formed even in generation of the oral cavity image only based on the CT data, this surface geometry data may be used in place of the oral cavity image based on the IOS data.

Data generation apparatus 1 may perform segmentation for each anatomical element shown in the oral cavity image, whether the oral cavity image is generated based on the IOS data or the CT data. For example, segmentation may be various, such as segmentation of each of a hard tissue and a soft tissue, segmentation of each of a maxillary hard tissue and a mandibular hard tissue, segmentation of each of the dentition and the alveolar bone, segmentation of each of the dentition in the maxilla and the dentition in the mandible, and segmentation of each of teeth. For example, data generation apparatus 1 may recognize an area of the dentition in the maxilla, the dentition in the mandible, each tooth included in the dentition in the maxilla, and each tooth included in the dentition in the mandible which are segmented by the user, segment each part of them, and show the part in the oral cavity image, based on the user input.

Alternatively, data generation apparatus 1 may automatically segment each part based on artificial intelligence (AI) technology, without obeying the user input. For example, data generation apparatus 1 may be provided with an estimation model including a neural network for identification of the dentition in the maxilla, the dentition in the mandible, and each of teeth shown in the oral cavity image, based on the oral cavity image data. The estimation model is trained in machine learning so as to segment the dentition in the maxilla, the dentition in the mandible, and each of teeth shown in the oral cavity image based on the inputted oral cavity image data, with training data in which a plurality of oral cavity images and ground truth data indicating an area of each segmented part shown in each of the plurality of oral cavity images are defined as a set. With such an estimation model, the user can obtain the oral cavity image showing the dentition in the maxilla, the dentition in the mandible, and each of teeth segmented by the estimation model of a control device 10, by inputting the oral cavity image data to data generation apparatus 1.

### [Configuration of Data Generation Apparatus]

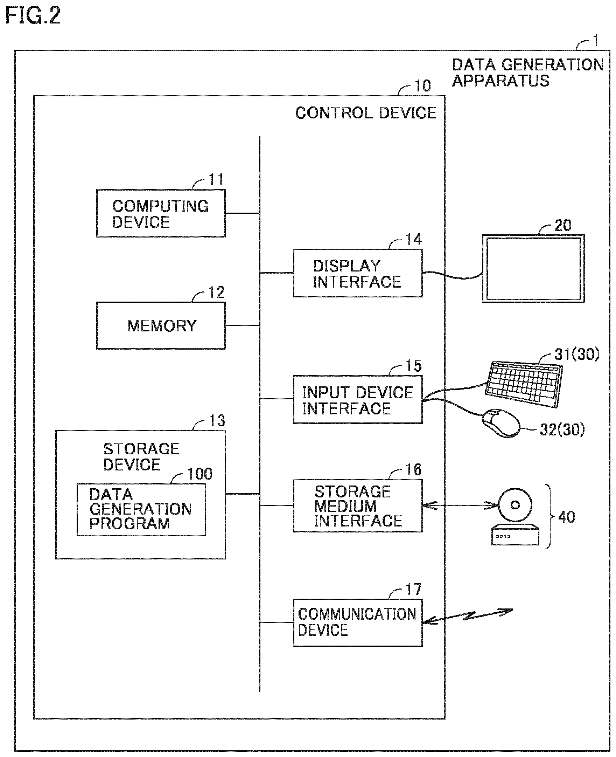

A configuration of data generation apparatus 1 according to the first embodiment will be described with reference to Fig. 2. Fig. 2 is a block diagram showing a configuration of data generation apparatus 1 according to the first embodiment. Data generation apparatus 1 may be implemented, for example, by a general-purpose computer or a dedicated computer.

As shown in Fig. 2, data generation apparatus 1 includes control device 10, display 20, and an input device 30.

Control device 10 includes a computing device 11, a memory 12, a storage device 13, a display interface 14, an input device interface 15, a storage medium interface 16, and a communication device 17.

Computing device 11 is a computing entity (computer) that performs various types of processing by executing various programs. Computing device 11 is implemented by a processor such as a central processing unit (CPU), a micro processing unit (MPU), a tensor processing unit (TPU), or a graphics processing unit (GPU). Though the processor which represents an exemplary computing device 11 performs functions to perform various types of processing by executing a program, some or all of these functions may be performed by dedicated hardware circuitry such as an application specific integrated circuit (ASIC) or a field programmable gate array (FPGA). The "processor" is not limited to a processor in a narrow sense that performs processing in accordance with a stored program architecture like the CPU, the MPU, the TPU, or the GPU, but may encompass hard-wired circuitry such as the ASIC or the FPGA. Computing device 11 is not limited to a von Neumann computer such as the CPU or the GPU but may be implemented by a non von Neumann computer such as a quantum computer or an optical computer. Computing device 11 as described above can also be read as processing circuitry that performs prescribed processing. Computing device 11 may be implemented by a single chip or a plurality of chips. Furthermore, the processor and relating processing circuitry may be implemented by a plurality of computers connected to one another through wires or wirelessly over a local area network or a wireless network. The processor and the relating processing circuitry may be implemented by a cloud computer that performs remote computation based on input data and outputs a result of computation to another device located at a remote position. Execution of various programs by computing device 11 also corresponds to various programs causing computing device 11 to perform various types of processing.

Memory 12 includes a volatile storage area (for example, a working area) where a program code or a work memory is temporarily stored in execution of various programs by computing device 11. Examples of memory 12 include a volatile memory such as a dynamic random access memory (DRAM) or a static random access memory (SRAM) or a non-volatile memory such as a read only memory (ROM) or a flash memory.

Various programs to be executed by computing device 11 or various types of data are stored in storage device 13. Storage device 13 may be implemented by one or more non-transitory computer readable media or one or more computer readable storage media. Examples of storage device 13 include a hard disk drive (HDD) and a solid state drive (SSD).

A data generation program 100 is stored in storage device 13. Contents of data generation processing for generation of oral cavity image data for checking by computing device 11, of an occlusal condition of a tooth are described in data generation program 100.

Display interface 14 is an interface for connection of display 20. Display interface 14 implements input and output of data between control device 10 and display 20. For example, control device 10 causes display 20 to show the oral cavity image based on oral cavity image data through display interface 14.

Input device interface 15 is an interface for connection of input device 30 such as a keyboard 31 and a mouse 32. Input device interface 15 implements input and output of data between control device 10 and input device 30. For example, the user can use input device 30 such as keyboard 31 and mouse 32 to input a desired command through input device interface 15, so as to have control device 10 generate and edit the oral cavity image data based on the command. Inputting by the user to control device 10 through input device 30 or data (command) inputted to control device 10 by the user is also referred to as the "user input."

Storage medium interface 16 reads various types of data stored in a storage medium 40 such as a removable disc or writes various types of data into storage medium 40. For example, data generation apparatus 1 may obtain data generation program 100 from storage medium 40 through storage medium interface 16 or write oral cavity image data for checking the occlusal condition of the tooth into storage medium 40 through storage medium interface 16. Storage medium 40 may be implemented by one or more non-transitory computer readable media or one or more computer readable storage media. Control device 10 may obtain the IOS data, the CT data, or combined data of the IOS data and the CT data from storage medium 40 through storage medium interface 16.

Communication device 17 transmits and receives data to and from an external apparatus through wired or wireless communication. For example, control device 10 may receive data generation program 100 from an external apparatus through communication device 17 or transmit oral cavity image data for checking the occlusal condition of the tooth to an external apparatus through communication device 17. Control device 10 may obtain the IOS data, the CT data, or combined data of the IOS data and the CT data from an external apparatus through communication device 17.

### [Exemplary Generation of Oral Cavity Image to Which Heat Map Is Annexed]

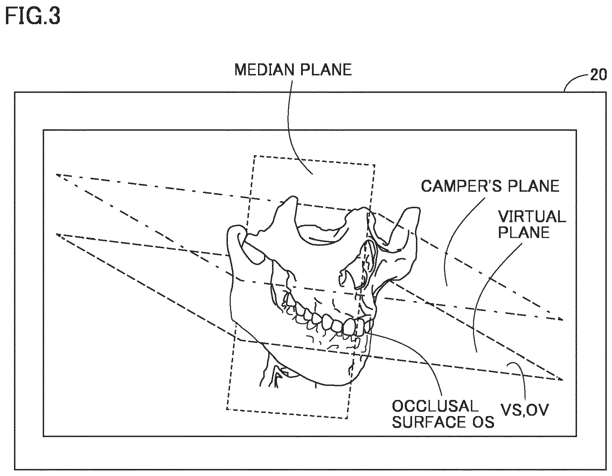

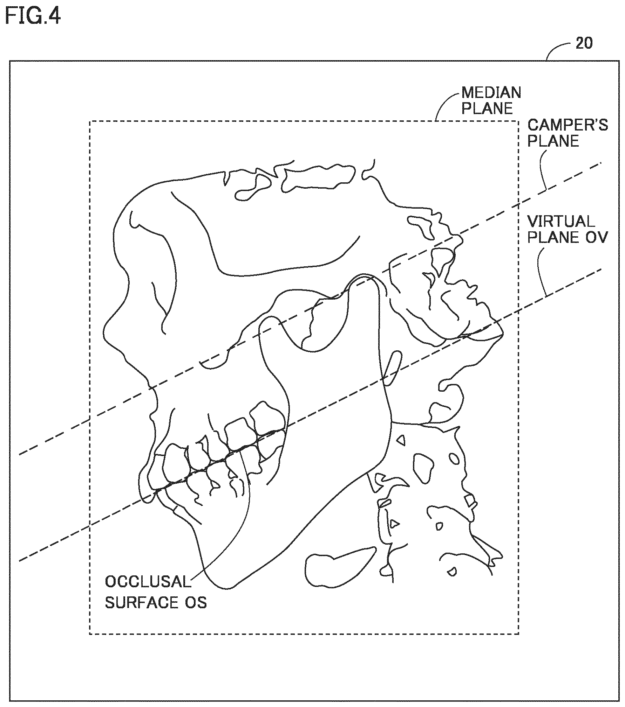

Exemplary generation by data generation apparatus 1 according to the first embodiment, of an oral cavity image to which a heat map is annexed will be described with reference to Figs. 3 to 7. Processing by data generation apparatus 1 which will be described below is performed by execution of data generation program 100 by control device 10 (computing device 11) of data generation apparatus 1. Figs. 3 and 4 are each a diagram for illustrating an exemplary virtual plane set in the oral cavity image.

As shown in Fig. 3, the user has the oral cavity image shown on display 20. The oral cavity image shown on display 20 shows the hard tissue portion such as the teeth and the bone. The oral cavity image may be an image showing the hard tissue portion including at least the teeth. The oral cavity image may be an image showing the hard tissue portion including at least a dental arch. The user uses input device 30 to superimpose a virtual plane on the oral cavity image. The virtual plane is an exemplary "virtual surface." For example, by moving or rotating the oral cavity image shown on display 20, the user can show on display 20 as shown in Fig. 3, the oral cavity image obtained when an imaging target is viewed from a diagonally front point of view or show on display 20 as shown in Fig. 4, the oral cavity image obtained when the imaging target is viewed from a point of view on a side surface side.

While the user views the oral cavity image, the user determines a position of the virtual plane and superimposes the virtual plane on the oral cavity image by placing the virtual plane at the determined position. In other words, the user can superimpose the virtual plane on the oral cavity image viewed from a specific point of view selected from among a plurality of points of view. In the examples in Figs. 3 and 4, the user superimposes the virtual plane on the oral cavity image, along occlusal surface OS where occlusion between the dentition in the maxilla and the dentition in the mandible is achieved. In other words, ideally, the user determines the position of the virtual plane such that the virtual plane coincides with occlusal surface OS. At this time, the user can set the virtual plane at a desired position while viewing the imaging target from various points of view by moving or rotating the oral cavity image. Furthermore, the user can also change at least one of a color and a transmittance of the virtual plane.

In the embodiment, a virtual surface set at a position of occlusal surface OS is also referred to as a "virtual surface VS." Virtual surface VS may be referred to as an occlusal position virtual surface OV. In an example where virtual surface VS is a plane, occlusal position virtual surface OV is an occlusal position virtual plane. In an example where virtual surface VS is a curved surface, occlusal position virtual surface OV is an occlusal position virtual curved surface. Occlusal position virtual surface OV may be set by an operation by the user or may automatically be set by image recognition, machine learning, or the like. Occlusal position virtual surface OV is set to be located in each of occlusal surfaces OS of teeth in a dental arch. Virtual surface VS, whether it is the plane or the curved surface, is preferably a continuous surface without fine projections and recesses so as to maintain overall uniformity for heat map observation. The virtual surface which means occlusal position virtual surface OV may be labeled with "OV". For example, the virtual plane which means occlusal position virtual surface OV may be expressed as a "virtual plane OV."

The user can also determine a position of a median plane and superimpose the median plane on the oral cavity image by placing the median plane at the determined position while viewing the oral cavity image. The median plane refers to a perpendicular plane (perpendicular when a human body stands erect) that passes through the median of the human body which is the imaging target shown in the oral cavity image, the plane dividing the human body into two parts when viewed from the front. Furthermore, the user can also determine a position of the Camper's plane and superimpose the Camper's plane on the oral cavity image by placing the Camper's plane at the determined position while viewing the oral cavity image. The Camper's plane refers to a plane defined by a line that connects a subnasale and tragions to one another. The Camper's plane may be defined by a plane defined by a line that connects points under left and right nasal wings and the external auditory meatus to one another. The user may determine as the position of the virtual plane (occlusal position virtual surface OV), a position that coincides with occlusal surface OS by translation of the Camper's plane downward (a direction toward the jaws, for example, the -Z direction) along the median plane.

Fig. 5 is a diagram for illustrating exemplary calculation of a Balkwill angle. As shown in Fig. 5, the Balkwill angle refers to an angle formed between occlusal surface OS and a Bonwill triangle. The Balkwill angle is normally within a range from 21 degrees to 31 degrees and an average of the Balkwill angle is 26 degrees. The Bonwill triangle refers to a triangle formed by a line that connects both of heads of temporomandibular joints and incisors to one another. The Bonwill triangle may be defined by a triangle formed by a line that connects both of tops of central portions of upper surfaces of mandibular condylar heads and an incisor point to one another.

After the user sets virtual plane OV along occlusal surface OS, the user can further set the Bonwill triangle described above and determine whether or not the Balkwill angle formed by the occlusal surface and the set Bonwill triangle is within a reference range (for example, a range from 21 degrees to 31 degrees) and whether or not the Balkwill angle deviates from a standard angle (for example, 26 degrees) by a prescribed value or more to thereby determine whether or not there is abnormality in jaws and occlusion of the subject. The user may set virtual plane (occlusal position virtual surface) OV such that the Balkwill angle is set to the standard angle (for example, 26 degrees). As shown in Fig. 5, though virtual plane OV is shown as a triangle having a small interior angle so as to facilitate understanding of use of the Bonwill triangle, it actually has an area wide to such an extent as covering the entire dental arch as shown in Fig. 3.

Virtual surface VS located at a position displaced from occlusal position virtual surface OV is also referred to as a displaced virtual surface TS. The virtual plane which means displaced virtual surface TS may be labeled with "TS" in the embodiment. For example, the virtual plane which means displaced virtual surface TS may be expressed as a "virtual plane TS." The virtual surface located at the position of occlusal position virtual surface OV may be considered as virtual plane TS an amount of displacement of which is zero. A direction of displacement of displaced virtual surface TS from occlusal position virtual surface OV may be a direction perpendicular to the virtual surface as described above if the virtual surface is the plane, or may be the -Z direction or the +Z direction. Displacement of displaced virtual surface TS is displacement including a component in a direction along the tooth axis. Such displacement is also referred to as "tooth axis direction component displacement." Tooth axis direction component displacement away from occlusal surface OS is also referred to as "displacement away from occlusion." Displacement toward occlusal surface OS from a position distant from occlusal surface OS is also referred to as "displacement toward occlusion." The direction including the component in the direction along the tooth axis is also referred to as a "tooth axis component direction." An amount of displacement in tooth axis direction component displacement is preferably uniform with respect to occlusal position virtual surface OV, at any three points not superimposed on a straight line on displaced virtual surface TS. A distance between each point on the surface of occlusal surface defining area OA and displaced virtual surface TS is also referred to as a "virtual surface separation distance VD." In the tooth axis component direction, a direction from occlusal surface OS toward a dental root may be considered as a down direction and a direction from the dental root toward the occlusal surface may be considered as an up direction. When a point A is located at a position toward the down direction as compared with a point B, point A may be considered as being located downstream from point B. When point A is located at a position toward the up direction as compared with point B, point A may be considered as being located upstream from point B. Since occlusal position virtual surface OV is set at a position on occlusal surface OS, displacement away from occlusion and displacement toward occlusion may be proximate separation from occlusal position virtual surface OV and approach to occlusal position virtual surface OV.

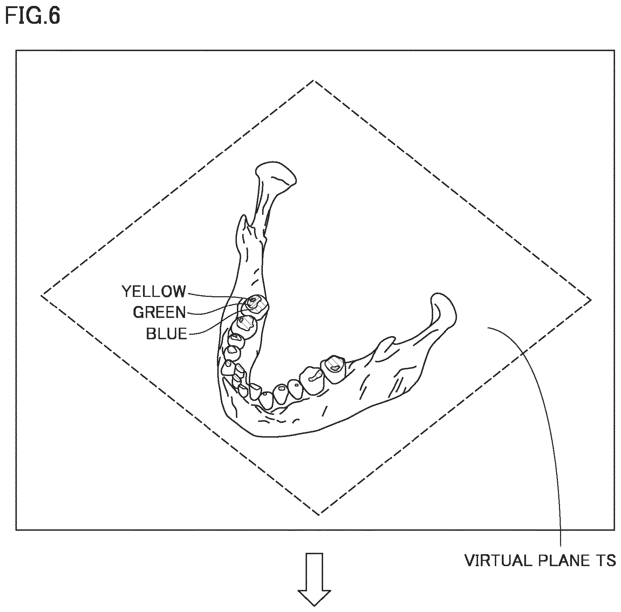

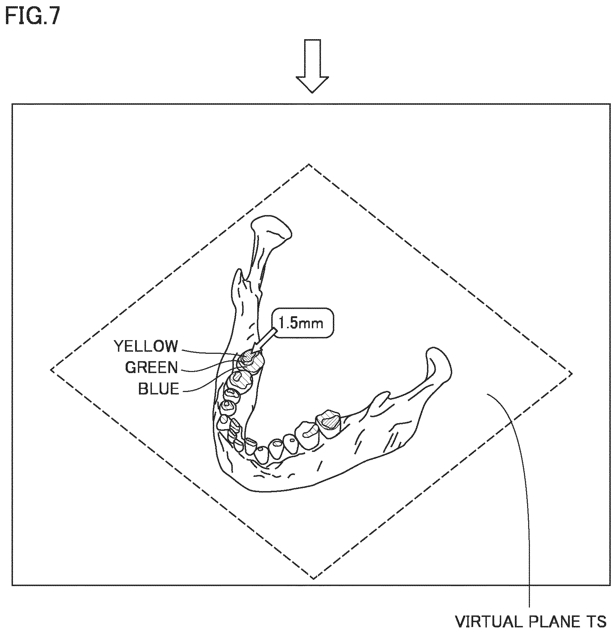

Figs. 6 and 7 are each a diagram for illustrating exemplary change of the heat map in the oral cavity image with movement of the virtual plane. The user can use input device 30 to drag virtual plane TS to move the position of virtual plane TS in a prescribed direction on the oral cavity image. For example, the user can move virtual plane TS in a direction perpendicular to virtual plane TS. As illustrated in Figs. 6 and 7, in an image showing one of the mandibular hard tissue and the maxillary hard tissue in the oral cavity image, the other of them does not have to be shown. When the user views the heat map on a side in which the user is more interested from a direction upward along the tooth axis or from a direction obliquely upward along the tooth axis in observation, visibility is better in a state where the other is not shown.

As the user moves virtual plane TS, distance VD between virtual plane TS and each point on occlusal surface (occlusal surface defining area) OA varies with movement of virtual plane TS; virtual plane TS is closer to each point on occlusal surface OA of each tooth or more distant from occlusal surface OS of each tooth. For example, when virtual plane TS set on occlusal surface OS moves in the -Z direction (toward the mandible), virtual plane TS and an unpassed point on virtual plane TS on occlusal surface OA of each tooth in the mandible are closer to each other, whereas virtual plane TS and occlusal surface OA of each tooth in the maxilla are more distant from each other. Alternatively, when virtual plane TS set on occlusal surface OA moves in the +Z direction (toward the maxilla), virtual plane TS and an unpassed point on virtual plane TS on occlusal surface OA of each tooth in the maxilla are closer to each other, whereas virtual plane TS and occlusal surface OA of each tooth in the mandible are more distant from each other.

Data generation apparatus 1 calculates distance VD between virtual plane TS moved based on the user input and occlusal surface OA of each tooth in the maxilla or the mandible shown in the oral cavity image, and changes the oral cavity image in accordance with the distance that varies depending on the position of the virtual plane. Specifically, data generation apparatus 1 calculates distance VD between each point that defines occlusal surface OA of each tooth shown in the oral cavity image and virtual plane TS and adds the heat map in accordance with distance VD to each point on occlusal surface OA.

For example, when the distance between the point that defines occlusal surface OA of the tooth and virtual plane TS is 0 mm in a prescribed direction (a direction perpendicular to the virtual plane), that is, when the point that defines occlusal surface OA is located on the virtual plane, data generation apparatus 1 adds a blue color to the point on the occlusal surface the distance from which is calculated. When the distance between the point that defines occlusal surface OA of the tooth and the virtual plane exceeds 0 mm and is shorter than 1.0 mm while the tooth protrudes from virtual plane TS in the prescribed direction (the direction perpendicular to the virtual plane, upward along the tooth axis here), data generation apparatus 1 adds a green color to the point on occlusal surface OA the distance from which is calculated. When the distance between the point (protruding point) that defines occlusal surface OA of the tooth and virtual plane TS is equal to or longer than 1.0 mm and shorter than 2.0 mm while the tooth protrudes from virtual plane TS in the prescribed direction (the direction perpendicular to the virtual plane, upward along the tooth axis here), data generation apparatus 1 adds a yellow color to the point (protruding point) on occlusal surface OA the distance from which is calculated. When the distance between the point (protruding point) that defines occlusal surface OA and virtual plane TS is equal to or longer than 2.0 mm and shorter than 3.0 mm while the tooth protrudes from virtual plane TS in the prescribed direction (the direction perpendicular to virtual plane TS, upward along the tooth axis here), data generation apparatus 1 adds a red color to the point (protruding point) on the occlusal surface the distance from which is calculated. When the distance between the point (protruding point) that defines occlusal surface OA and virtual plane TS is equal to or longer than 3.0 mm while the tooth protrudes from virtual plane TS in the prescribed direction (the direction perpendicular to virtual plane TS, upward along the tooth axis here), data generation apparatus 1 does not add a color to the point (protruding point) on occlusal surface OA the distance from which is calculated. Data generation apparatus 1 may provide heat map representation of the oral cavity image by using a color designated by the user, based on the user input.

Various types of allocation of the color in the heat map are applicable, and for example, different types of allocation as below are also applicable.

### (Different Type of Allocation 1)

No color is added when the tooth protrudes from virtual plane TS in the prescribed direction (the direction perpendicular to virtual plane TS, upward along the tooth axis here). When the distance between the point that defines occlusal surface OA of the tooth and virtual plane TS is 0 mm, the red color is added to the point on occlusal surface OA the distance from which is calculated. When the distance between the point that defines occlusal surface OA of the tooth and virtual plane TS exceeds 0 mm and is shorter than 1.0 mm while the tooth is distant from virtual plane TS in the prescribed direction (the direction perpendicular to virtual plane TS, downward along the tooth axis here), the yellow color is added to the point on occlusal surface OA the distance from which is calculated. When the distance between the point that defines occlusal surface OA of the tooth and virtual plane TS is equal to or longer than 1.0 mm and shorter than 2.0 mm while the tooth is distant from virtual plane TS in the prescribed direction (the direction perpendicular to virtual plane TS, downward along the tooth axis here), the green color is added to the point on occlusal surface OA the distance from which is calculated. When the distance between the point that defines occlusal surface OA of the tooth and virtual plane TS is equal to or longer than 2.0 mm and shorter than 3.0 mm while the tooth is distant from virtual plane TS in the prescribed direction (the direction perpendicular to virtual plane TS, downward along the tooth axis here), the blue color is added to the point on occlusal surface OA the distance from which is calculated.

### (Different Type of Allocation 2)

When the distance between the point (protruding point) that defines occlusal surface OA of the tooth and virtual plane TS is equal to or longer than 1.0 mm while the tooth protrudes from virtual plane TS in the prescribed direction (the direction perpendicular to virtual plane TS, upward along the tooth axis here), the red color is added to the point on occlusal surface OA the distance from which is calculated. When the distance between the point (protruding point) that defines occlusal surface OA of the tooth and virtual plane TS is equal to or longer than 0 mm and shorter than 1.0 mm while the tooth protrudes from virtual plane TS in the prescribed direction (the direction perpendicular to virtual plane TS, upward along the tooth axis here), an orange color is added to the point on occlusal surface OA the distance from which is calculated. When the distance between the point that defines occlusal surface OA of the tooth and virtual plane TS is 0 mm, the yellow color is added to the point on occlusal surface OA the distance from which is calculated. When the distance between the point that defines occlusal surface OA of the tooth and virtual plane TS exceeds 0 mm and is shorter than 1.0 mm while the tooth is distant from virtual plane TS in the prescribed direction (the direction perpendicular to virtual plane TS, downward along the tooth axis here), the green color is added to the point on occlusal surface OA the distance from which is calculated. When the distance between the point that defines occlusal surface OA of the tooth and virtual plane TS is equal to or longer than 1.0 mm while the tooth is distant from virtual plane TS in the prescribed direction (the direction perpendicular to virtual plane TS, downward along the tooth axis here), the blue color is added to the point on occlusal surface OA the distance from which is calculated.

As described above, since data generation apparatus 1 can show the distance of each point that defines occlusal surface OA of each tooth from virtual plane TS in the heat map in the oral cavity image, the user can readily check the premature contact position of each tooth.

For example, as the user moves virtual plane TS from the position of the virtual plane shown in Fig. 6 toward the mandible, the heat map added to each point on occlusal surface OA changes with the distance between each point that defines occlusal surface OA of each tooth in the mandible and virtual plane TS as shown in Fig. 7. For example, with attention being paid to a yellow-colored area in the heat map shown in Fig. 7 and the heat map shown in Fig. 6, an amount of displacement away from occlusion is larger in the state in Fig. 7 than in the state in Fig. 6 and an area where the amount is not smaller than 1.0 mm and smaller than 2.0 mm has increased, and hence a colored area has increased.

Since the user can thus check in which order which position on occlusal surface OA of each tooth comes in contact with virtual plane TS while the user moves virtual plane TS, the user can highly accurately check the occlusal condition of the tooth.

Furthermore, data generation apparatus 1 can set a spot designated by the user on occlusal surface OA of the tooth shown in the oral cavity image based on the user input, and add a numerical value in accordance with the distance between the point that defines occlusal surface OA of the tooth and virtual plane TS to the set spot. For example, as shown in Fig. 7, when the user designates a desired spot on occlusal surface OA of a desired tooth by moving a cursor with the use of input device 30, data generation apparatus 1 provides pop-up representation of the numerical value (in the example in Fig. 7, "1.5 mm") indicating the distance between the point corresponding to the spot and virtual plane TS around the designated spot. The user can thus readily check the occlusal condition at the designated spot.

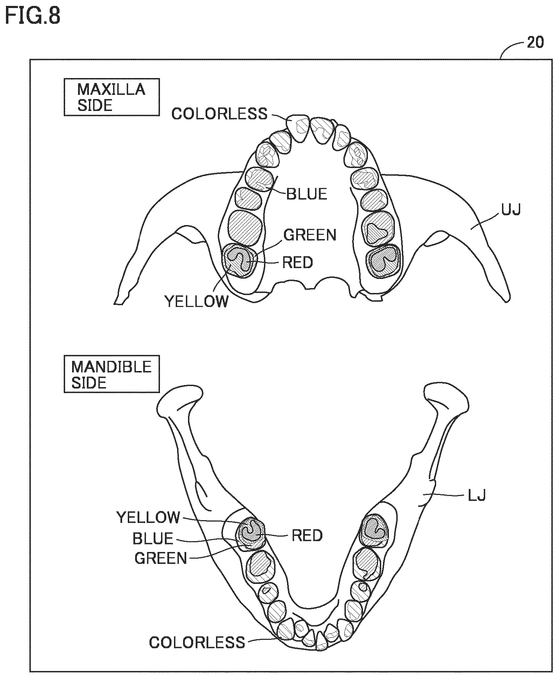

Fig. 8 is a diagram for illustrating an example of the dentition in the maxilla and the dentition in the mandible to which the heat map is annexed. As shown in Fig. 8, data generation apparatus 1 can show on display 20, occlusal surface OA of the tooth to which the heat map is added, separately for the dentition in the maxilla and the dentition in the mandible.

For example, data generation apparatus 1 can add the heat map to each of the dentition in the maxilla and the dentition in the mandible with the dentition being developed and capture occlusal surface OA of each of the dentition in the maxilla and the dentition in the mandible to which the heat map is added, and thus the data generation apparatus can store in storage device 13 as a still image, a state of occlusal surface OA of each of the dentition in the maxilla and the dentition in the mandible to which the heat map is added.

### [Processing by Data Generation Apparatus]

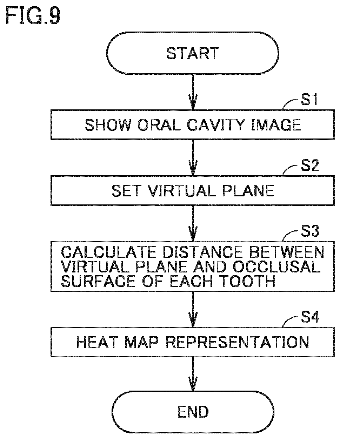

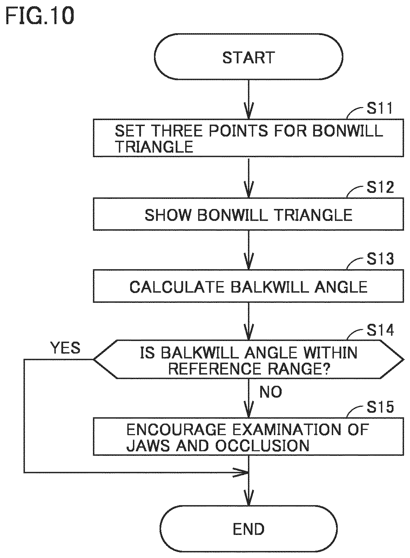

Processing performed by data generation apparatus 1 according to the first embodiment will be described with reference to Figs. 9 and 10. Fig. 9 is a flowchart for illustrating exemplary data generation processing performed by data generation apparatus 1 according to the first embodiment. Each STEP (which is denoted as "S" below) shown in Figs. 9 and 10 is performed by execution of data generation program 100 by control device 10 (computing device 11) of data generation apparatus 1.

As shown in Fig. 9, data generation apparatus 1 shows on display 20, the oral cavity image three-dimensionally showing the oral cavity including the tooth (S1). For example, data generation apparatus 1 shows on display 20, the oral cavity image showing the two-dimensional hard tissue portion (the bone, the tooth, or the like) viewed from a prescribed point of view as illustrated in Fig. 3. At this time, data generation apparatus 1 may segment the dentition in the maxilla, the dentition in the mandible, and each of teeth shown in the oral cavity image based on the user input. Alternatively, data generation apparatus 1 may identify the dentition in the maxilla, the dentition in the mandible, and each of teeth shown in the oral cavity image with the use of an estimation model trained in machine learning or the like and show each part of them in the oral cavity image as being segmented. Processing in S1 is an exemplary "display step."

Data generation apparatus 1 superimposes the virtual plane on the oral cavity image shown on display 20, based on the user input provided through input device 30 (S2). For example, as illustrated in Fig. 3, data generation apparatus 1 superimposes the virtual plane on the oral cavity image, along the occlusal surface where occlusion between the dentition in the maxilla and the dentition in the mandible is achieved, based on the user input. At this time, data generation apparatus 1 shows on display 20, the oral cavity image viewed from a plurality of points of view and superimposes virtual surface (occlusal position virtual surface) OV on the oral cavity image viewed from a specific point of view selected from among the plurality of points of view, based on the user input. Furthermore, data generation apparatus 1 may change at least one of the color and the transmittance of the virtual surface based on the user input. Processing in S2 is an exemplary "image superimposition step."

Data generation apparatus 1 calculates the distance between the virtual plane and the occlusal surface of each tooth (S3). For example, data generation apparatus 1 calculates the distance between the virtual plane fixed at the prescribed position and each point that defines the occlusal surface of each tooth in the maxilla or the mandible shown in the oral cavity image.

Data generation apparatus 1 adds the heat map to the oral cavity image shown on display 20, in accordance with the calculated distance between the virtual surface and the occlusal surface, specifically in accordance with the distance between the virtual surface and each point that defines the occlusal surface of each tooth (S4). For example, as illustrated in Fig. 6, data generation apparatus 1 adds a predetermined color in accordance with the distance between the virtual surface and each point that defines the occlusal surface of each tooth to each point. Thereafter, data generation apparatus 1 quits the present process. As illustrated in Fig. 7, when the virtual plane is moved based on the user input, data generation apparatus 1 can calculate again the distance between the moved virtual plane and each point that defines the occlusal surface of each tooth, and add the heat map to the oral cavity image shown on display 20, that is, change the oral cavity image, in accordance with the calculated distance. Processing in S3 and S4 is an exemplary "image change step."

Suitably, in S2, as an initial operation, data generation apparatus 1 initially accepts a position setting operation to set a position of occlusal position virtual surface OV so as to set the position of occlusal position virtual surface OV. As described above, data generation apparatus 1 may automatically set the position of occlusal position virtual surface OV. Furthermore, in S2, data generation apparatus 1 accepts a position setting operation to set a position of displaced virtual surface TS so as to set the position of displaced virtual surface TS. In S3, data generation apparatus 1 calculates virtual surface separation distance VD. In S4, data generation apparatus 1 adds the heat map. At this time, data generation apparatus 1 returns to processing following S1, and as processing before S2, it determines whether it has accepted the position setting operation to set the position of displaced virtual surface TS at another position. When the position setting operation is not performed, data generation apparatus 1 quits present processing, whereas when the position setting operation is performed, data generation apparatus 1 sets new displaced virtual surface TS in S2. After data generation apparatus 1 sets new displaced virtual surface TS, the process proceeds to S3 where data generation apparatus 1 calculates virtual surface separation distance VD, and the process proceeds to S4 where data generation apparatus 1 adds the heat map. While a movement operation continues, loop processing as described above is repeated. Data generation apparatus 1 may proceed to S3 and S4 also in a stage of setting occlusal position virtual surface OV to add the heat map where the amount of displacement is zero.

Further suitably, data generation apparatus 1 may determine whether or not displaced virtual surface TS set in S2 is located downstream from occlusal position virtual surface OV. When displaced virtual surface TS is located downstream from occlusal position virtual surface OV, data generation apparatus 1 calculates virtual surface separation distance VD in S3 and adds the heat map in S4. When displaced virtual surface TS is located upstream from occlusal position virtual surface OV, however, the process does not have to proceed to S3 or later, or even when the process proceeds to step S3, the process does not have to proceed to S4. Further suitably, the heat map may be added to occlusal surface (occlusal surface defining area) OA upstream from displaced virtual surface TS and does not have to be added to occlusal surface (occlusal surface defining area) OA downstream from displaced virtual surface TS.

Namely, data generation apparatus 1 may be configured as below. In the image change step, the position of the virtual surface is moved in the prescribed direction based on the user input, the mouth cavity image is changed in accordance with the distance that changes depending on the position of the virtual surface, and the heat map in accordance with the distance is added to the occlusal surface of the tooth shown in the mouth cavity image.

When an area composed of an area including occlusal surface OS or an area including occlusal surface OS and an area contiguous to occlusal surface OS is defined as occlusal surface defining area OA, the virtual surface located at the occlusal position of the tooth is defined as occlusal position virtual surface OV, and the virtual surface obtained by displacement of occlusal position virtual surface OV is defined as displaced virtual surface TS, when the direction including the component in the direction along the tooth axis is defined as the tooth axis component direction, in the tooth axis component direction, the direction from the occlusal surface toward the dental root is defined as the down direction, the direction from the dental root toward the occlusal surface is defined as the up direction, the position toward the down direction is defined as downstream, and the position toward the up direction is defined as upstream, and when the distance between each point on the surface of occlusal surface defining area OA and displaced virtual surface TS is defined as virtual surface separation distance VD, the virtual surface to be moved is displaced virtual surface TS obtained by movement of the virtual surface located at the position of occlusal position virtual surface OV in the tooth axis component direction. When displaced virtual surface TS is located downstream from occlusal position virtual surface OV, data generation apparatus 1 calculates virtual surface separation distance VD and adds the heat map to occlusal surface defining area OA, and when displaced virtual surface TS is located upstream from occlusal position virtual surface OV, data generation apparatus 1 does not add the heat map at least to occlusal surface defining area OA. Furthermore, data generation apparatus 1 adds the heat map to occlusal surface defining area OA upstream from displaced virtual surface TS but does not add the heat map to occlusal surface defining area OA downstream from displaced virtual surface TS.

As shown in Fig. 8, data generation apparatus 1 may provide heat map representation of a maxilla image UJ and a mandible image LJ on the screen of display 20. In the example in Fig. 8, maxilla image UJ is shown in an upper portion of the screen and mandible image LJ is shown in a lower portion of the screen. Furthermore, in each of maxilla image UJ and mandible image LJ, individual displaced virtual surface TS may be set. Though an operation for axial direction component displacement may individually be performed on individual displaced virtual surface TS, the amount of displacement in axial direction component displacement of displaced virtual surface TS in maxilla image UJ and the amount of displacement in axial direction component displacement of displaced virtual surface TS in mandible image LJ may be equal to each other. For example, when data generation apparatus 1 receives an operation for displacement away from occlusion in the down direction in maxilla image UJ, it may automatically apply displacement away from occlusion the same in amount of displacement also to mandible image LJ.

As illustrated in Fig. 8, data generation apparatus 1 may capture the occlusal surface of each of the dentition in the maxilla and the dentition in the mandible in the oral cavity image to which the heat map is added, based on the user input, and store in storage device 13 as a still image, the state of the occlusal surface of each of the dentition in the maxilla and the dentition in the mandible to which the heat map is added. Furthermore, as illustrated in Fig. 7, data generation apparatus 1 may set the spot designated by the user on the occlusal surface of the tooth shown in the oral cavity image based on the user input and provide pop-up representation of a numerical value in accordance with the distance between the point that defines the occlusal surface of the tooth and the virtual plane for the set spot. Data generation apparatus 1 may output the numerical value in accordance with the distance between the point that defines the occlusal surface of the tooth and the virtual plane in a comma separated values (CSV) file or a text file.

As set forth above, data generation apparatus 1 according to the first embodiment can superimpose the virtual plane on the oral cavity image shown on display 20 based on the user input and change the heat map in the oral cavity image in accordance with the distance between the virtual plane and the occlusal surface of the tooth shown in the oral cavity image. Since the user can thus highly accurately check the occlusal condition of the tooth with the use of the oral cavity image shown on display 20 to which the heat map is annexed, the user can readily know which part of the dentition in the maxilla and the dentition in the mandible should be treated to adjust bite.

Fig. 10 is a flowchart for illustrating exemplary Balkwill angle determination processing performed by the data generation apparatus according to the first embodiment. Data generation apparatus 1 can determine whether or not the Balkwill angle of a subject is appropriate by performing processing shown in Fig. 10.

As shown in Fig. 10, data generation apparatus 1 sets three points for the Bonwill triangle in the oral cavity image shown on display 20 (S11). For example, as illustrated in Fig. 5, data generation apparatus 1 sets three points for the Bonwill triangle in both of heads of temporomandibular joints and the incisors based on the user input. Data generation apparatus 1 superimposes the Bonwill triangle on the oral cavity image by connecting the three points for the Bonwill triangle with a line and show the oral cavity image on display 20 (S12).

Data generation apparatus 1 calculates the Balkwill angle formed between the occlusal surface or the virtual plane set along the occlusal surface and the Bonwill triangle (S13). Data generation apparatus 1 determines whether or not the calculated Balkwill angle is within the reference range (S14). For example, data generation apparatus 1 determines whether or not the Balkwill angle is within the range from 21 degrees to 31 degrees. Alternatively, data generation apparatus 1 determines whether or not the Balkwill angle deviates from the standard angle (for example, 26 degrees) by a prescribed value or more. Data generation apparatus 1 can set any reference range (for example, from 21 degrees to 31 degrees) or any standard angle (for example, 26 degrees) based on the user input.

When the Balkwill angle is within the reference range (YES in S14), data generation apparatus 1 quits the present process. When the Balkwill angle is not within the reference range (NO in S14), on the other hand, there may be abnormality in the jaws and occlusion of the subject and hence data generation apparatus 1 shows a message encouraging examination of the jaws and occlusion on display 20 (S15). Thereafter, data generation apparatus 1 quits the present process.

As set forth above, data generation apparatus 1 can determine whether or not the Balkwill angle of the subject is normal, and when the Balkwill angle is not normal, the data generation apparatus can encourage the subject to undergo the examination of the jaws and occlusion.

### <Second Embodiment>

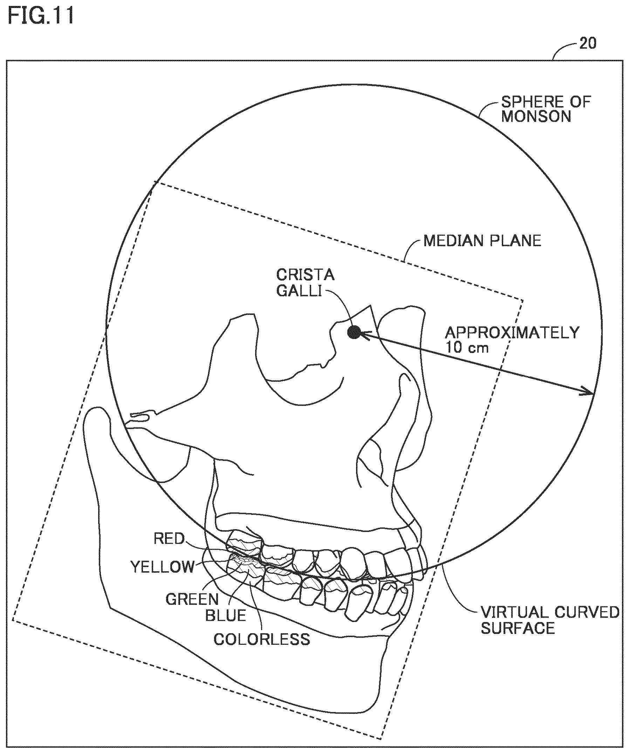

Data generation apparatus 1 according to a second embodiment will be described with reference to Figs. 11 to 13. Processing in data generation apparatus 1 according to the second embodiment which will be described below is performed by execution of data generation program 100 by control device 10 (computing device 11) of data generation apparatus 1. Only a difference of data generation apparatus 1 according to the second embodiment from data generation apparatus 1 according to the first embodiment will be described, and a component identical to that of data generation apparatus 1 according to the first embodiment has the same reference character allotted and description thereof will not be repeated.

Though data generation apparatus 1 according to the first embodiment described above superimposes the virtual plane as an exemplary "virtual surface" on the oral cavity image, data generation apparatus 1 according to the second embodiment may superimpose a virtual curved surface obtained by making use of a sphere of Monson on the oral cavity image, by way of example of the "virtual surface." Fig. 11 is a diagram for illustrating an exemplary sphere of Monson set in the oral cavity image.

As shown in Fig. 11, the sphere of Monson is a sphere having a radius of approximately 10 cm around the crista galli included in the ethmoid. It has theoretically been said that a spherical surface of such a sphere of Monson passes through the mandibular head, the incisal edge, and cusps, and mandibular motion is made along the spherical surface of the sphere of Monson. In addition, extension of the tooth axis orthogonal to the occlusal surface of each tooth has been said to pass through the center of the sphere of Monson. A curve that extends upward in terms of an upward/downward direction of the head when cusps (the cusp of the canine tooth in the mandible, the cusps on a cheek side of premolar teeth and molar teeth, the entire surface of the mandibular ramus, and the foreground of the mandibular head) are aligned in a lateral view of the dentition in the mandible is also referred to as a curve of Spee. When occlusion is normal, the curve of Spee is substantially flat or slightly arc. When occlusion is abnormal, however, the curve of Spee is great. Such a curve of Spee has been said to pass over the spherical surface of the sphere of Monson.

The user can use input device 30 to superimpose the virtual curved surface as described above on the oral cavity image. For example, while the user views the oral cavity image from any point of view, the user sets a central position of the sphere of Monson and sets a diameter or a radius of the sphere of Monson with the set central position being defined as the reference. For example, as shown in Fig. 11, the user superimposes the spherical surface (the curve of Spee) of the sphere of Monson on the oral cavity image, along the occlusal surface where occlusion between the dentition in the maxilla and the dentition in the mandible is achieved. In other words, the user ideally determines the position of the virtual curved surface such that the virtual curved surface coincides with the occlusal surface. At this time, the user can set the virtual curved surface at a desired position while viewing the imaging target from various points of view by moving or rotating the oral cavity image. Furthermore, the user can change at least one of the color and the transmittance of the virtual curved surface.

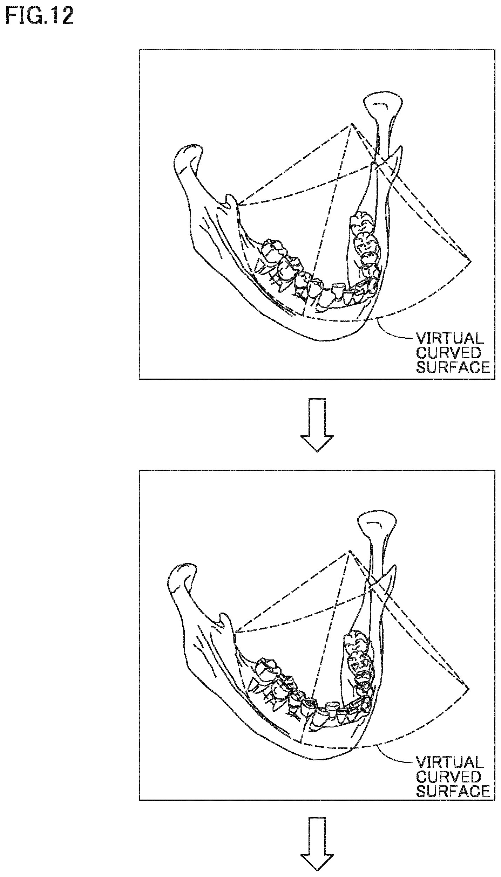

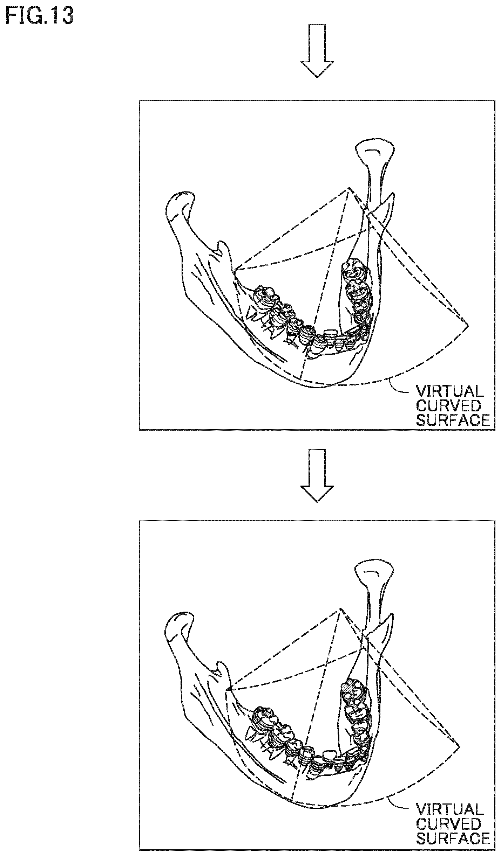

Figs. 12 and 13 are each a diagram for illustrating exemplary change of the heat map in the oral cavity image with movement of the virtual curved surface. A partial surface of the sphere, a curved surface defined by a partial surface of the sphere of Monson here, is defined as the virtual curved surface. The user can use input device 30 to drag the virtual curved surface to move a position thereof in a prescribed direction over the oral cavity image. Specifically, data generation apparatus 1 moves a central position of the sphere of Monson along a prescribed plane set in the oral cavity image, specifically along the median plane, toward the occlusal surface, based on the user input. Data generation apparatus 1 thus changes the heat map, that is, changes the oral cavity image, in accordance with the distance between the virtual surface and the occlusal surface that changes depending on the central position of the sphere (sphere of Monson).

As the user moves the virtual curved surface, with the movement of the virtual curved surface, the virtual curved surface is closer to or more distant from the occlusal surface of each tooth. For example, when the virtual curved surface set on the occlusal surface is moved downward (toward the mandible), the virtual curved surface and each tooth in the mandible are closer to each other, whereas the virtual curved surface and the occlusal surface of each tooth in the maxilla are more distant from each other. When the virtual curved surface set on the occlusal surface is moved upward (toward the maxilla), the virtual curved surface and each tooth in the maxilla are closer to each other, whereas the virtual curved surface and the occlusal surface of each tooth in the mandible are more distant from each other.

Data generation apparatus 1 calculates the distance between the virtual curved surface moved based on the user input and the occlusal surface of each tooth in the maxilla or the mandible shown in the oral cavity image, and changes the oral cavity image in accordance with the distance that changes depending on the position of the virtual curved surface. Specifically, data generation apparatus 1 calculates the distance between each point that defines the occlusal surface of each tooth shown in the oral cavity image and the virtual curved surface and adds the heat map in accordance with the distance to each point on the occlusal surface.

For example, when the user moves the virtual curved surface from the side of the maxilla toward the mandible as shown in Figs. 12 and 13, the heat map added to each point on the occlusal surface changes with the distance between each point that defines the occlusal surface of each tooth in the mandible and the virtual curved surface. In the example shown in Figs. 12 and 13, with movement of the virtual curved surface, a colored area in the heat map annexed to the oral cavity image becomes larger stepwise.

Since the user can thus check in which order which position on the occlusal surface of each tooth comes in contact with the virtual curved surface while the user moves the virtual curved surface, the user can highly accurately check the occlusal condition of the tooth.

Similarly to data generation apparatus 1 according to the first embodiment illustrated in Fig. 7, data generation apparatus 1 according to the second embodiment may set a spot designated by the user on the occlusal surface of the tooth shown in the oral cavity image and add a numerical value in accordance with the distance between the point that defines the occlusal surface of the tooth and the virtual curved surface to the set spot. Furthermore, similarly to data generation apparatus 1 according to the first embodiment illustrated in Fig. 8, data generation apparatus 1 according to the second embodiment may show on display 20, the occlusal surface of the tooth to which the heat map is added, separately for the dentition in the maxilla and the dentition in the mandible.

As set forth above, data generation apparatus 1 according to the second embodiment can superimpose the virtual curved surface on the oral cavity image shown on display 20 based on the user input and change the heat map in the oral cavity image in accordance with the distance between the virtual curved surface and the occlusal surface of the tooth shown in the oral cavity image. Since the user can thus highly accurately check the occlusal condition of the tooth with the use of the oral cavity image shown on display 20 to which the heat map is annexed, the user can readily know which part of the dentition in the maxilla and the dentition in the mandible should be treated to adjust bite.

### <Modification>

The present disclosure is not limited to examples above, but can variously be modified and applied. Modifications applicable to the present disclosure will be described below.

Though data generation apparatus 1 is configured to change the heat map annexed to the oral cavity image in accordance with the distance between the virtual surface and the occlusal surface of the tooth shown in the oral cavity image, it may change the oral cavity image with another method. For example, data generation apparatus 1 may show on display 20, a numerical value indicating the distance between the virtual surface and the occlusal surface of the tooth shown in the oral cavity image and change the numerical value shown on display 20 with change in distance. Alternatively, data generation apparatus 1 may change a pattern provided to the occlusal surface of each tooth shown in the oral cavity image or a transmittance of each tooth, in accordance with the distance between the virtual surface and the occlusal surface of the tooth shown in the oral cavity image. Alternatively, data generation apparatus 1 may show the heat map with a grayscale.

Though data generation apparatus 1 is configured to move the virtual plane in the direction perpendicular to the virtual plane or to move the central position of the sphere of Monson along the median plane set in the oral cavity image based on the user input, it may be configured to move the virtual surface in any direction desired by the user based on the user input.

Though data generation apparatus 1 is configured to set the virtual surface manually designated by the user, it may automatically set the virtual surface based on the AI technology or the like, without obeying the user input. For example, data generation apparatus 1 (control device 10) may be provided with an estimation model for estimation based on the oral cavity image, of the position of the virtual surface to be superimposed on the oral cavity image. The estimation model is trained in machine learning to estimate an appropriate position of the virtual surface to be superimposed on the oral cavity image based on inputted oral cavity image data, with training data where a plurality of oral cavity images and ground truth data indicating the position of the virtual surface to be superimposed on each of the plurality of oral cavity images are defined as a set. With the use of such an estimation model, the user can achieve automatic superimposition of the virtual surface on a position estimated by the estimation model of control device 10 by inputting oral cavity image data to data generation apparatus 1. Furthermore, data generation apparatus 1 may be configured to move and adjust the virtual surface automatically set as such to a position desired by the user.

The configuration of data generation apparatus 1 described above may be provided in X-ray equipment (CT imager). For example, the X-ray equipment includes an imaging unit to image the maxilla and the mandible of a subject. The X-ray equipment may obtain CT data representing the hard tissue portion (the bone, the teeth, or the like) around the maxilla and the mandible of the subject by imaging by the imaging unit, generate the oral cavity image based on the obtained CT data, and show the oral cavity image on display 20. Furthermore, the X-ray equipment may superimpose the virtual surface on the oral cavity image shown on display 20 based on the user input and change the heat map added to the oral cavity image in accordance with the distance between the virtual surface and the occlusal surface of the tooth shown in the oral cavity image.

It should be understood that the embodiments disclosed herein are illustrative and non-restrictive in every respect. The scope of the present disclosure is defined by the terms of the claims rather than the description above and is intended to include any modifications within the scope and meaning equivalent to the terms of the claims. The configuration illustrated in the present embodiments and the configuration illustrated in the modifications can be combined as appropriate.

### REFERENCE SIGNS LIST

1 data generation apparatus; 10 control device; 11 computing device; 12 memory; 13 storage device; 14 display interface; 15 input device interface; 16 storage medium interface; 17 communication device; 20 display; 30 input device; 31 keyboard; 32 mouse; 40 storage medium; 100 data generation program.

## Claims

1. A data generation program configured to generate image data for checking a state of a tooth, the data generation program configured to cause a computer to perform:
a display step of showing on a display, an oral cavity image three-dimensionally showing an oral cavity including the tooth;
an image superimposition step of superimposing a virtual surface on the oral cavity image based on a user input or machine learning; and
an image change step of changing the oral cavity image in accordance with a distance between the virtual surface and an occlusal surface of the tooth shown in the oral cavity image.

2. The data generation program according to claim 1, wherein
the image change step includes causing, based on the user input, the computer to move a position of the virtual surface in a prescribed direction and to change the oral cavity image in accordance with the distance that changes depending on the position of the virtual surface.

3. The data generation program according to claim 2, wherein
the prescribed direction is a direction perpendicular to the virtual surface.

4. The data generation program according to claim 1 or 2, wherein
the image superimposition step includes causing, based on the user input, the computer to show on the display, the oral cavity image viewed from a plurality of points of view and to superimpose the virtual surface on the oral cavity image viewed from a specific point of view selected from among the plurality of points of view.

5. The data generation program according to claim 1 or 2, being configured to further cause the computer to change at least one of a color and a transmittance of the virtual surface based on the user input.

6. The data generation program according to claim 1 or 2, wherein
the image change step includes causing the computer to add a heat map in accordance with the distance to the occlusal surface of the tooth shown in the oral cavity image.

7. The data generation program according to claim 6, being configured to further cause the computer to show on the display, the occlusal surface of the tooth to which the heat map is added, separately for a dentition in a maxilla and a dentition in a mandible.

8. The data generation program according to claim 1 or 2, being configured to further cause, based on the user input, the computer to set a spot designated by a user on the occlusal surface of the tooth shown in the oral cavity image and to add a numerical value in accordance with the distance to the spot.

9. The data generation program according to claim 1 or 2, wherein
the virtual surface is a plane or a curved surface.

10. The data generation program according to claim 9, wherein
the curved surface is defined by a partial surface of a sphere set in the oral cavity image, and
the data generation program is configured to further cause the computer to set a central position and a diameter or a radius of the sphere based on the user input.

11. The data generation program according to claim 10, wherein
the image change step includes causing, based on the user input, the computer to move the central position of the sphere along a prescribed plane set in the oral cavity image and to change the oral cavity image in accordance with the distance that changes depending on the central position of the sphere.

12. The data generation program according to claim 1 or 2, wherein
the oral cavity image is generated based on at least one of optical scanner data obtained by an optical scanner, the optical scanner data including position information of each point in a point group indicating a surface of the tooth, and CT data obtained by computed tomography (CT) scan of the tooth.

13. The data generation program according to claim 1 or 2, being configured to further cause the computer to perform segmentation for each anatomical element shown in the oral cavity image.

14. A data generation method of generating image data for checking a state of a tooth, the data generation method comprising, as processing to be performed by a computer:
a display step of showing on a display, an oral cavity image three-dimensionally showing an oral cavity including the tooth;
an image superimposition step of superimposing a virtual surface on the oral cavity image based on a user input or machine learning; and
an image change step of changing the oral cavity image in accordance with a distance between the virtual surface and an occlusal surface of the tooth shown in the oral cavity image.

15. A data generation apparatus configured to generate image data for checking a state of a tooth, the data generation apparatus comprising:
a display configured to show an image;
an input device configured to receive a user input; and
a control device configured to control the display based on the user input, wherein
the control device is configured to
cause the display to show an oral cavity image three-dimensionally showing an oral cavity including the tooth,
superimpose a virtual surface on the oral cavity image based on the user input or machine learning, and
change the oral cavity image in accordance with a distance between the virtual surface and an occlusal surface of the tooth shown in the oral cavity image.

16. X-ray equipment configured to generate image data for checking a state of a tooth, the X-ray equipment comprising:
an imaging unit configured to image an oral cavity including the tooth;
a display configured to show an image;
an input device configured to receive a user input; and
a control device configured to control the display based on the user input, wherein
the control device is configured to
cause the display to show an oral cavity image three-dimensionally showing the oral cavity imaged by the imaging unit,
superimpose a virtual surface on the oral cavity image based on the user input or machine learning, and
change the oral cavity image in accordance with a distance between the virtual surface and an occlusal surface of the tooth shown in the oral cavity image.
